(19) Europäisches Patentamt
European Patent Office
Office européen des brevets

(11) Veröffentlichungsnummer: **0 311 907 B1**

# (12) EUROPÄISCHE PATENTSCHRIFT

(45) Veröffentlichungstag der Patentschrift: **21.07.93**

(51) Int. Cl.[5]: **C07D 213/30**, C07D 213/89, A01N 43/40

(21) Anmeldenummer: **88116553.4**

(22) Anmeldetag: **06.10.88**

## (54) 3-substituierte Pyridine und diese enthaltende Fungizide.

(30) Priorität: **14.10.87 DE 3734750**

(43) Veröffentlichungstag der Anmeldung:
**19.04.89 Patentblatt 89/16**

(45) Bekanntmachung des Hinweises auf die Patenterteilung:
**21.07.93 Patentblatt 93/29**

(84) Benannte Vertragsstaaten:
**AT BE CH DE ES FR GB GR IT LI NL SE**

(56) Entgegenhaltungen:
**EP-A- 0 117 485**
**EP-A- 0 214 566**
**CH-A- 498 568**
**US-A- 4 710 508**

(73) Patentinhaber: **BASF Aktiengesellschaft**
**Carl-Bosch-Strasse 38**
**W-6700 Ludwigshafen(DE)**

(72) Erfinder: **Zipperer, Bernhard, Dr.**
**Am Herrgottsacker 6**
**W-6716 Dirmstein(DE)**
Erfinder: **Goetz, Norbert, Dr.**
**Schoefferstrasse 25**
**W-6520 Worms 1(DE)**
Erfinder: **Buschmann, Ernst, Dr.**
**Georg Ludwig Krebs-Strasse 10**
**W-6700 Ludwigshafen(DE)**
Erfinder: **Sproesser, Linhard, Dr.**
**Goethestrasse 4**
**W-6702 Bad Duerkheim(DE)**
Erfinder: **Ammermann, Eberhard, Dr.**
**Sachsenstrasse 3**
**W-6700 Ludwigshafen(DE)**
Erfinder: **Lorenz, Gisela, Dr.**
**Erlenweg 13**
**W-6730 Neustadt(DE)**

EP 0 311 907 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die vorliegende Erfindung betrifft neue 3-substituierte Pyridinderivate sowie fungizide Mittel, die diese Verbindungen enthalten, und Verfahren zur Bekämpfung von Pilzen.

Strukturell verwandte 3-Propenylpyridine, z.B. 2-n-Butyl-3-(4-methylphenyl)-1-(3-pyridinyl)-2-propen-1-ol, 2-n-Butyl-3-(4-methylphenyl)-1-(3-pyridinyl)-2-propen-1-on, 2-n-Butyl-3-(4-fluorphenyl)-1-(3-pyridinyl)-2-propen-1-ol sind aus EP-214 566 als Fungizide bekannt. Ihre Wirkung ist jedoch in bestimmten Indikationsbereichen, insbesondere bei niedrigen Aufwandmengen, nicht immer ganz befriedigend.

Es wurde nun gefunden, daß 3-substituierte Pyridinderivate der Formel

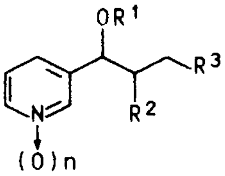 (I),

in der

$R^1$ Wasserstoff, $C_1$-$C_8$-Alkyl, $C_3$-$C_8$-Alkenyl, $C_3$-$C_8$-Alkinyl, $C_2$-$C_{12}$-Acyl, $C_2$-$C_{12}$-Halogenacyl, ungesättigtes $C_3$-Acyl, gegebenenfalls ein- bis dreimal durch die Reste $C_1$-$C_4$-Alkyl, $C_1$-$C_4$-Alkoxy, Phenyl, Acetoxy, $C_1$-$C_4$-Halogenalkyl, Halogen, Nitro substituiertes Benzoyl, gegebenenfalls durch 1 bis 3 Reste des Typs $C_1$-$C_4$-Alkyl, $C_1$-$C_4$-Alkoxy, $C_1$-$C_4$-Halogenalkyl, Halogen, Cyan, Nitro substituiertes Benzyl,

$R^2$ Wasserstoff, $C_1$-$C_{12}$-Alkyl, $C_3$-$C_{12}$-Cycloalkyl, $C_4$-$C_{20}$-Cycloalkylalkyl, $C_4$-$C_{20}$-Alkylcycloalkyl,

$R^3$ den Rest

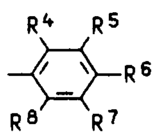 oder 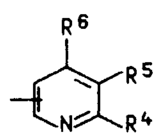 bedeutet, wobei

$R^4$ bis $R^8$ unabhängig voneinander Wasserstoff, $C_1$-$C_4$-Alkyl, $C_1$-$C_4$-Alkoxy, $C_1$-$C_4$-Halogenalkyl, $C_2$-$C_4$-Alkoxycarbonyl, $C_2$-$C_4$-Dialkylamino, Phenyl, Hydroxy oder Halogen bedeuten und $R^4$ und $R^5$ oder $R^5$ und $R^6$ gemeinsam zusätzlich die Gruppe -CH = CH-CH = CH- bedeuten, n den Wert 0 oder 1 hat und ihre für Pflanzen verträglichen Salze überraschend eine deutlich bessere fungizide Wirkung aufweisen als die ungesättigten Pyridinderivate aus EP-214 566.

$R^1$ bedeutet beispielsweise Wasserstoff, Methyl, Ethyl, n-Propyl, Isopropyl, n-Butyl, Isobutyl, tert.-Butyl, n-Pentyl, Isopentyl, n-Hexyl, neo-Hexyl, n-Heptyl, n-Octyl, 2,4,4-Trimethylpent-2-yl, 2-Ethyl-hexyl; Allyl, 2-Methylallyl, 3-Methylallyl, 3,3-Dimethylallyl, 3-Buten-1-yl, 3-Octen-1-yl; Propargyl, 3-Octin-1-yl; Acetyl, Mono-, Di- oder Trichloracetyl, Trifluoracetyl, Propionyl, 3-Brompropionyl, Butyryl, 4-Brombutyryl, 3-Methylbutyryl, 3,3-Dimethylbutyryl, Pentanoyl, Hexanoyl, Heptanoyl, Octanoyl, 2-Ethylhexanoyl, Nonanoyl, Decanoyl, Lauroyl, Dodecanoyl, Acryloyl, Benzoyl, Mono-, Di-oder Trimethylbenzoyl, 4-tert.-Butylbenzoyl, 4-Phenylbenzoyl, Fluorbenzoyl, Mono-, Di- oder Trichlorbenzoyl, Trifluormethylbenzoyl, Mono-, Di- oder Trimethoxybenzoyl, 2-Acetoxybenzoyl, Mono- oder Dinitrobenzoyl; Benzyl, Mono- Di- oder Trimethylbenzyl, 4-tert.-Butylbenzyl, Mono-, Di- oder Trimethoxybenzyl, Trifluormethylbenzyl, Trichlormethylbenzyl, Mono-, Di- oder Trichlorbenzyl, Cyanbenzyl, Mono- oder Dinitrobenzyl.

$R^2$ bedeutet beispielsweise Wasserstoff; Methyl, Ethyl, n-Propyl, Isopropyl, n-Butyl, Isobutyl, tert.-Butyl, n-Pentyl, Isopentyl, neo-Pentyl, n-Hexyl, neo-Hexyl, n-Heptyl, n-Octyl, n-Nonyl, n-Decyl, n-Undecyl, n-Dodecyl; Cyclopentyl, Cyclohexyl, Cyclooctyl, Cyclododecyl; 2-Cyclohexylethyl, 3-Cyclohexyl-2-methyl-propyl; 4-tert.-Butylcyclohexyl;

$R^3$ bedeutet beispielsweise Phenyl, Mono-, Di- oder Trimethylphenyl, Ethylphenyl, 4-tert.-

Butylphenyl, Mono-, Di- oder Trimethoxyphenyl, Trifluormethylphenyl, Trichlormethylphenyl, N,N-Dimethyl(ethyl)aminophenyl, Methoxycarbonylphenyl, Hydroxyphenyl, Biphenyl, Mono-, Di-oder Trichlorphenyl, Fluorphenyl, Naphthyl, Pyridyl, Methylpyridyl, Chlorpyridyl, Chinolinyl, Isochinolinyl.

Für Pflanzen verträgliche Salze sind z. B. Säureadditions-Salze mit anorganischen Mineralsäuren wie Hydrochloride, Hydrobromide, Sulfate, Phosphate, Nitrate; Salze mit Ameisensäure oder mit Alkylcarbonsäuren wie Acetate, 2-Ethylhexanoate, Oxalate; Salze mit Arylsulfonsäuren, wie Benzolsulfonate, Toluolsulfonate, Dodecylbenzolsulfonate.

Die Verbindungen der Formel I können zwei Asymmetrie-Zentren aufweisen und daher in zwei diastereomeren Formen vorliegen, die ggf. durch bekannte Verfahren getrennt werden können. Sowohl die einzelnen Diastereomeren als auch ihre Mischungen werden von der Erfindung umfaßt.

Die Verbindungen der Formel I können in Form der Pyridine oder in Form der Pyridin-N-Oxide vorliegen.

Die neuen 3-substituierten Pyridine der Formel I können z.B. hergestellt werden, indem man Pyridylalkohole der Formel I,

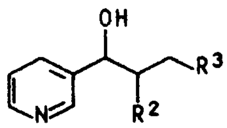

(I),

in der $R^2$ und $R^3$ die oben genannten Bedeutungen haben, umsetzt mit einem Alkylierungs- oder Acylierungsmittel der Formel II,

$R^1$-X (II),

in der $R^1$ die oben genannten Bedeutungen außer Wasserstoff hat und X eine nucleofuge Gruppe, z. B. Chlor, Brom, Jod, Methansulfonat, Benzolsulfonat, p-Toluolsulfonat bedeutet.

Zweckmäßig wird bei dieser Umsetzung so vorgegangen, daß ein Gemisch aus Pyridylalkohol der Formel I, mindestens äquimolarer Menge einer Hilfsbase und einem inerten, organischen Lösungsmittel wie Diethylether, Tetrahydrofuran, Dioxan, Dimethylformamid, Dimethylsulfoxid, Acetonitril, Toluol, Xylol vorgelegt und das Alkylierungs- oder Acylierungsmittel zudosiert wird.

Die Reaktionstemperaturen betragen 0° bis 120°C, vorzugsweise 20°C bis 80°C.

Als Hilfsbasen können anorganische und organische Säurebindemittel verwendet werden.

Beispiele für anorganische Basen sind Natriumhydrid, Natriumamid, Natriumhydroxid, Natriumcarbonat, Kaliumcarbonat, Kaliumhydroxid.

Beispiele für organische Basen sind Amine, insbesondere tertiäre Amine wie Triethylamin, Ethyldiisopropylamin, Pyridin und Alkoholate wie Natriummethylat, Natriumethylat, Kalium-tert.-butylat.

In einer vorteilhaften Variante dieses Verfahrens werden die Umsetzungen der Pyridylalkohole der Formel I mit Alkylierungsmitteln der Formel II in einem Zweiphasensystem, bestehend aus wässriger Natronlauge und einem organischen Lösungsmittel, vorzugsweise Toluol oder Dichlormethan, unter Zusatz eines Phasentransferkatalysators, z.B. Tetra-n-butylammoniumchlorid, Benzyltriethylammoniumchlorid, Methyltrioctylammoniumchlorid umgesetzt.

Die Herstellung der neuen Verbindungen I, wobei $R^1$ Wasserstoff bedeutet, erfolgt z.B. in der Weise, daß man die Pyridylketone der Formel III oder die Pyridylalkohole der Formel IV,

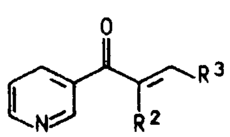

(III)

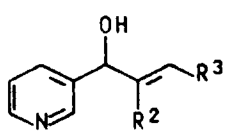

(IV)

in denen $R^2$ und $R^3$ die oben genannten Bedeutungen haben, mit einem Reduktionsmittel, z. B. einem komplexen Hydrid oder Wasserstoff in Gegenwart eines Katalysators reduziert (vgl. z. B. M. Hudlicky, Reductions in Organic Chemistry, S. 119 f., Ellis Horwood Series, Chichester, 1984). Die Herstellung der Pyridylketone der Formel III und der Pyridylalkohole der Formel IV ist bekannt (EP-214 566).

Ein weiteres Verfahren zur Herstellung der neuen Verbindungen I, wobei $R^1$ Wasserstoff bedeutet, besteht z.B. darin, daß man Organometallverbindungen der Formel V

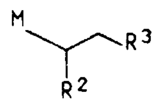

in der $R^2$ und $R^3$ die oben genannten Bedeutungen haben und M für Lithium oder die Reste MgCl oder MgBr steht, mit Nicotinaldehyd (3-Formylpyridin) umsetzt. Zweckmäßig wird hierzu die Organometallverbindung der Formel V in einem inerten Lösungsmittel, vorzugsweise Diethylether oder Tetrahydrofuran, vorgelegt und der Nicotinaldehyd im Gemisch mit demselben Lösungsmittel bei -30 °C bis +50 °C zudosiert.

Die metallorganischen Verbindungen der Formel V sind nach bekannten Verfahren aus den entsprechenden Halogeniden (Halogen = Chlor, Brom) zugänglich (vgl, z.B. Houben-Weyl, Methoden der Organischen Chemie, Band 13/1, S. 134 f., Georg Thieme Verlag, Stuttgart, 1970; ibid., Band 13/2a, S. 54 f., Georg Thieme Verlag, Stuttgart, 1973). Letztere sind entweder bekannt oder können nach bekannten Verfahren hergestellt werden. Die Pyridin-N-Oxide erhält man aus den Pyridinen nach bekannten Verfahren, z.B. durch Oxidation mit Persäuren (vgl. z.B. Katritzky, Lagowski, Chemistry of Heterocyclic N-Oxides, S. 21 bis 56, Academie Press, New York, 1971).

Die folgenden Beispiele erläutern die Herstellung der neuen fungiziden Pyridine:

Beispiel 1

2-n-Butyl-3-(4-fluorphenyl)-1-(3-pyridinyl)-propan-1-ol

In einem 0,3 l Rührautoklaven wird eine Mischung aus 17,1 g (0,6 mol) 2-n-Butyl-3-(4-fluorphenyl)-1-(3-pyridinyl)-2-propen-1-ol, 160 ml Tetrahydrofuran und 4,5 g Hydrierkontakt (0,5 % Pd auf $Al_2O_3$) bei 30 °C und 50 bar Wasserstoffdruck bis zur Druckkonstanz hydriert. Die Lösung wird danach über Kieselgel abgesaugt, das Filtrat i. Vak. eingeengt und der Rückstand destillativ gereinigt. Ausbeute 7,9 g der Verbindung Nr. 1, Sdp. 187 °C (0,3 mbar).

Beispiel 2

2-n-Butyl-3-(4-fluorphenyl)-1-(3-pyridinyl)-propan-1-ol-2,4-dichlorbenzoat

Zur Lösung von 5,74 g (0,02 mol) Verbindung Nr. 1 in 50 ml Dichlormethan und 10 ml Triethylamin tropft man bei Raumtemperatur 4,6 g (0,022 mol) 2,4-Dichlorbenzoylchlorid gelöst in 10 ml Dichlormethan. Man rührt über Nacht, gibt dann 30 ml 10proz. $NaHCO_3$-Lösung zu und rührt nochmals eine Stunde. Die organ. Phase wird mit Wasser gewaschen, über $MgSO_4$ getrocknet und i. Vak. eingeengt. Der Rückstand wird chromatographisch gereinigt (Kieselgel; Laufmittel: Dichlormethan/Aceton 9:1). 6,7 g (73% d.T.) Verbindung Nr. 2 als gelbes Öl.

Beispiel 3

2-n-Butyl-3-(4-fluorphenyl)-1-(3-pyridinyl)-propan-1-ol-4-chlor-benzyl-ether

Eine Lösung von 5,74 g (0,02 mol) Verbindung 1 in 20 ml Dimethylformamid wird mit 0,72 g (0,03 mol) Natriumhydrid versetzt und bis zur Beendigung der Wasserstoffentwicklung bei 50 °C gerührt. Man kühlt auf Raumtemperatur und tropft dann 4,0 g (0,025 mol) 4-Chlorbenzylchlorid in 10 ml Dimethylformamid so langsam zu, daß die Innentemperatur nicht über 40 °C steigt. Man rührt eine Stunde bei Raumtemperatur (20 °C) nach, tropft bei 0 °C vorsichtig 50 l Wasser zu und versetzt mit 100 ml Ether. Die wäßrige Phase wird abgetrennt und mit Ether ausgeschüttelt. Die vereinigten organischen Phasen werden über $MgSO_4$ getrocknet und i. Vak. eingeengt. Der Rückstand wird chromatographisch gereinigt (Kieselgel; Laufmittel Dichlormethan/Aceton 9:1). 6,7 g (81 % d.Th.) Verbindung Nr. 3 als gelbes Öl.

Beispiel 4

3-(4-Fluorphenyl)-1-(3-pyridinyl)-propan-1-ol

Zu einer Lösung hergestellt aus 2,92 g (0,12 mol) Magnesium und 24,4 g (0,12 mol) 2-(4-Fluorphenyl)-ethylbromid in 250 ml Diethylether tropft man unter Rühren die Lösung von 10,7 g (0,10 mol) Nicotinaldehyd in 100 ml Diethylether und kocht anschließend zwei Stunden unter Rückfluß. Nach dem Abkühlen wird mit Wasser hydrolysiert und mit gesättigter wäßriger $NH_4Cl$-Lösung pH = 8 eingestellt. Die Phasen werden getrennt, die wäßrige Phase wird noch zweimal mit Ether extrahiert, die vereinigten organischen Phasen werden mit Wasser gewaschen und über $MgSO_4$ getrocknet. Nach Abdampfen des Lösungsmittels wird i.Vak. destilliert. 10,2 g (44 % d.Th.) Verbindung Nr. 4 vom Sdp. 190 °C (2 mbar).

Die folgenden Verbindungen werden in entsprechender Weise erhalten.

Tabelle 1 n = 0

| Bsp.-Nr. | $R^1$ | $R^2$ | $R^3$ | Kp/Fp |
|---|---|---|---|---|
| 1 | H | n-Butyl | 4-Fluorphenyl | 187°C (0,3 mbar) |
| 2 | 2,4-Dichlorbenzoyl | n-Butyl | 4-Fluorphenyl | Öl |
| 3 | 4-Chlorbenzyl | n-Butyl | 4-Fluorphenyl | Öl |
| 4 | H | H | 4-Fluorphenyl | 190° (2 mbar) |
| 5 | H | n-Butyl | 4-Methylphenyl | 167° (0,1 mbar) |
| 6 | H | n-Butyl | 4-tert.-Butylphenyl | 197°C (0,25 mbar) |
| 7 | H | n-Butyl | 4-Chlorphenyl | 196-200°C (0,2 mbar) |
| 8 | H | n-Butyl | 2,4-Dichlorphenyl | |
| 9 | H | n-Butyl | 4-Methoxyphenyl | |
| 10 | H | n-Butyl | 3-Trifluormethylphenyl | |
| 11 | H | n-Butyl | 4-Biphenyl | |
| 12 | H | n-Butyl | 2-Chlorphenyl | |
| 13 | H | n-Butyl | 1-Naphthyl | |
| 14 | H | n-Butyl | 2-Naphthyl | |
| 15 | H | n-Butyl | 3-Pyridinyl | |
| 16 | H | neo-Pentyl | 4-Fluorphenyl | |
| 17 | H | neo-Pentyl | 4-Chlorphenyl | |
| 18 | H | neo-Pentyl | 2-Chlorphenyl | |
| 19 | H | neo-Pentyl | 2,4-Dichlorphenyl | |
| 20 | H | n-Heptyl | 2,4-Dichlorphenyl | |
| 21 | H | n-Heptyl | 2-Chlorphenyl | |
| 22 | H | n-Heptyl | 4-Chlorphenyl | |
| 23 | H | n-Heptyl | 4-Fluorphenyl | |
| 24 | H | n-Undecyl | 2,4-Dichlorphenyl | |

| Bsp.-Nr. | $R^1$ | $R^2$ | $R^3$ | Kp/Fp |
|---|---|---|---|---|
| 38 | Acetyl | n-Butyl | 4-Fluorphenyl | |
| 39 | Acetyl | n-Butyl | 4-tert.-Butylphenyl | |
| 40 | Trifluoracetyl | n-Butyl | 4-tert.-Butylphenyl | |
| 41 | H | H | 4-Chlorphenyl | 200° C (2 mbar) |
| 42 | H | H | 2-Naphthyl | 230° (1 mbar) |
| 43 | H | $CH_3$ | Phenyl | 170° (1 mbar) |
| 44 | H | H | 2-Methylphenyl | 190° (2 mbar) |
| 60 | H | n-Propyl | Phenyl | |
| 61 | H | n-Propyl | 4-Chlorphenyl | |
| 62 | H | n-Propyl | 2-Chlorphenyl | |
| 63 | H | n-Propyl | 2,4-Dichlorphenyl | |

| Bsp.-Nr. | $R^1$ | $R^2$ | $R^3$ | Kp/Fp |
|---|---|---|---|---|
| 64 | H | n-Propyl | 4-Fluorphenyl | |
| 65 | H | n-Propyl | 4-Methylphenyl | |
| 66 | H | n-Propyl | 4-tert.-Butylphenyl | |
| 67 | H | iso-Propyl | Phenyl | |
| 68 | H | iso-Propyl | 4-Chlorphenyl | |
| 69 | H | iso-Propyl | 2-Chlorphenyl | |
| 70 | H | iso-Propyl | 2,4-Dichlorphenyl | |
| 71 | H | iso-Propyl | 4-Fluorphenyl | |
| 72 | H | iso-Propyl | 4-tert.-Butylphenyl | |
| 73 | H | Ethyl | Phenyl | |
| 74 | H | Ethyl | 4-Chlorphenyl | |
| 75 | H | Ethyl | 2-Chlorphenyl | |
| 76 | H | Ethyl | 2,4-Dichlorphenyl | |
| 77 | H | Ethyl | 4-Fluorphenyl | |
| 78 | H | Ethyl | 4-tert.-Butylphenyl | |
| 79 | H | Ethyl | 4-Methylphenyl | |
| 80 | H | Methyl | 4-Chlorphenyl | |
| 81 | H | Methyl | 2-Chlorphenyl | |
| 82 | H | Methyl | 2,4-Dichlorphenyl | |
| 83 | H | Methyl | 4-Fluorphenyl | |
| 84 | H | Methyl | Phenyl | |
| 85 | H | Methyl | 4-tert.-Butylphenyl | |
| 86 | H | Methyl | 4-iso-Propylphenyl | |
| 87 | H | Methyl | 4-Methoxyphenyl | |

Tabelle 2 n = 1

| Bsp.-Nr. | $R^1$ | $R^2$ | $R^3$ | Fp |
|---|---|---|---|---|
| 45 | H | n-Butyl | 4-Fluorphenyl | |
| 46 | H | n-Butyl | 4-Methylphenyl | |
| 47 | H | n-Butyl | 4-tert.-Butylphenyl | |
| 48 | H | n-Butyl | 4-Chlorphenyl | 128-130°C |
| 49 | H | n-Butyl | 2,4-Dichlorphenyl | |
| 50 | H | n-Butyl | 2-Chlorphenyl | |
| 51 | H | n-Butyl | 1-Naphthyl | |
| 52 | H | neo-Pentyl | 4-Fluorphenyl | |
| 53 | H | neo-Pentyl | 4-Chlorphenyl | |
| 54 | H | n-Heptyl | 4-Fluorphenyl | |
| 55 | H | n-Heptyl | 4-Chlorphenyl | |
| 56 | H | n-Heptyl | 2,4-Dichlorphenyl | |
| 57 | H | n-Undecyl | 4-Fluorphenyl | |
| 58 | H | n-Undecyl | 4-Chlorphenyl | |
| 59 | H | n-Undecyl | 2,4-Dichlorphenyl | |
| 88 | H | n-Propyl | 4-Chlorphenyl | |
| 89 | H | n-Propyl | 2,4-Dichlorphenyl | |
| 90 | H | n-Propyl | 2-Chlorphenyl | |
| 91 | H | n-Propyl | 4-Fluorphenyl | |
| 92 | H | iso-Propyl | 4-Chlorphenyl | |
| 93 | H | iso-Propyl | 2,4-Dichlorphenyl | |
| 94 | H | iso-Propyl | 2-Chlorphenyl | |
| 95 | H | iso-Propyl | 4-Fluorphenyl | |
| 96 | H | Ethyl | 4-Chlorphenyl | |
| 97 | H | Ethyl | 2,4-Dichlorphenyl | |

| Bsp.-Nr. | $R^1$ | $R^2$ | $R^3$ | Kp/Fp |
|---|---|---|---|---|
| 98 | H | Ethyl | 2-Chlorphenyl | |
| 99 | H | Ethyl | 4-Fluorphenyl | |
| 100 | H | Methyl | 4-Chlorphenyl | |
| 101 | H | Methyl | 2,4-Dichlorphenyl | |
| 102 | H | Methyl | 2-Chlorphenyl | |
| 103 | H | Methyl | 4-Fluorphenyl | |

Die neuen Verbindungen zeichnen sich, allgemein ausgedrückt, durch eine hervorragende Wirksamkeit gegen ein breites Spektrum von pflanzenpathogenen Pilzen, insbesondere aus der Klasse der Ascomyceten und Basidiomyceten, aus. Sie sind zum Teil systemisch wirksam und können als Blatt- und Bodenfungizide eingesetzt werden.

Besonders interessant sind die fungiziden Verbindungen für die Bekämpfung einer Vielzahl von Pilzen an verschiedenen Kulturpflanzen oder ihren Samen, insbesondere Weizen, Roggen, Gerste, Hafer, Reis,

Mais, Rasen, Baumwolle, Soja, Kaffee, Zuckerrohr, Obst und Zierpflanzen im Gartenbau, Weinbau sowie Gemüse - wie Gurken, Bohnen und Kürbisgewächse -.

Die neuen Verbindungen sind insbesondere geeignet zur Bekämpfung folgender Pflanzenkrankheiten:

Erysiphe graminis (echter Mehltau) in Getreide,
Erysiphe cichoracearum und Sphaerotheca fuliginea an Kürbisgewächsen,
Podosphaera leucotricha an Äpfeln,
Uncinula necator an Reben,
Puccinia-Arten an Getreide,
Rhizoctonia-Arten an Baumwolle und Rasen,
Ustilago-Arten an Getreide und Zuckerrohr,
Venturia inaequalis (Schorf) an Äpfeln,
Helminthosporium-Arten an Getreide,
Septoria nodorum an Weizen,
Botrytis cinerea (Grauschimmel) an Erdbeeren, Reben,
Cercospora arachidicola an Erdnüssen,
Pseudocercosporella herpotrichoides an Weizen, Gerste,
Pyricularia oryzae an Reis,
Phytophthora infestans an Kartoffeln und Tomaten,
Fusarium- und Verticillium-Arten an verschiedenen Pflanzen,
Plasmopara viticola an Reben,
Alternaria-Arten an Gemüse und Obst.

Die Verbindungen werden angewendet, indem man die Pflanzen mit den Wirkstoffen besprüht oder bestäubt oder die Samen der Pflanzen mit den Wirkstoffen behandelt. Die Anwendung erfolgt vor oder nach der Infektion der Pflanzen oder Samen durch die Pilze.

Die neuen Substanzen können in die üblichen Formulierungen übergeführt werden, wie Lösungen, Emulsionen, Suspensionen, Stäube, Pulver, Pasten und Granulate. Die Anwendungsformen richten sich ganz nach den Verwendungszwecken; sie sollen in jedem Fall eine feine und gleichmäßige Verteilung der wirksamen Substanz gewährleisten. Die Formulierungen werden in bekannter Weise hergestellt, z.B. durch Verstrecken des Wirkstoffs mit Lösungsmitteln und/oder Trägerstoffen, gegebenenfalls unter Verwendung von Emulgiermitteln und Dispergiermitteln, wobei im Falle der Benutzung von Wasser als Verdünnungsmittel auch andere organische Lösungsmittel als Hilfslösungsmittel verwendet werden können. Als Hilfsstoffe kommen dafür im wesentlichen in Frage: Lösungsmittel wie Aromaten (z.B. Xylol), chlorierte Aromaten (z.B. Chlorbenzole), Paraffine (z.B. Erdölfraktionen), Alkohole (z.B. Methanol, Butanol), Ketone (z.B. Cyclohexanon), Amine (z.B. Ethanolamin, Dimethylformamid) und Wasser; Trägerstoffe wie natürliche Gesteinsmehle (z.B. Kaoline, Tonerden, Talkum, Kreide) und synthetische Gesteinsmehle (z.B. hochdisperse Kieselsäure, Silikate); Emulgiermittel, wie nichtionogene und anionische Emulgatoren (z.B. Polyoxyethylen-Fettalkohol-Ether, Alkylsulfonate und Arylsulfonate) und Dispergiermittel, wie Lignin, Sulfitablaugen und Methylcellulose.

Die fungiziden Mittel enthalten im allgemeinen zwischen 0,1 und 95, vorzugsweise zwischen 0,5 und 90 Gew.% Wirkstoff.

Die Aufwandmengen liegen je nach Art des gewünschten Effektes zwischen 0,02 und 3 kg Wirkstoff oder mehr je ha. Die neuen Verbindungen können auch im Materialschutz eingesetzt werden, z.B. gegen Paecilomyces variotii.

Die Mittel bzw. die daraus hergestellten gebrauchsfertigen Zubereitungen, wie Lösungen, Emulsionen, Suspensionen, Pulver, Stäube, Pasten oder Granulate werden in bekannter Weise angewendet, beispielsweiise durch Versprühen, Vernebeln, Verstäuben, Verstreuen, Beizen oder Gießen.

Beispiele für solche Zubereitungen sind:

I. Man vermischt 90 Gew.-Teile der Verbindung Nr. 1 mit 10 Gew.-Teilen N-Methyl-$\alpha$-pyrrolidon und erhält eine Lösung, die zur Anwendung in Form kleinster Tropfen geeignet ist.

II. 20 Gew.-Teile der Verbindung Nr. 5 werden in einer Mischung gelöst, die aus 80 Gew.-Teilen Xylol, 10 Gew.-Teilen des Anlagerungsproduktes von 8 bis 10 Mol Ethylenoxid an 1 Mol Ölsäure-N-monoethanolamid, 5 Gew.-Teilen Calciumsalz der Dodecylbenzolsulfonsäure und 5 Gew.-Teilen des Anlagerungsproduktes und 40 Mol Ethylenoxid an 1 Mol Ricinusöl besteht. Durch Ausgießen und feines Verteilen der Lösung in Wasser erhält man eine wäßrige Dispersion.

III. 20 Gew.-Teile der Verbindung Nr. 6 werden in einer Mischung gelöst, die aus 40 Gew.-Teilen Cyclohexanon, 30 Gew.-Teilen Isobutanol, 20 Gew.-Teilen des Anlagerungsproduktes von 40 Mol Ethylenoxid an 1 Mol Ricinusöl besteht. Durch Eingießen und feines Verteilen der Lösung in Wasser erhält man eine wäßrige Dispersion.

IV. 20 Gew.-Teile der Verbindung Nr. 1 werden in einer Mischung gelöst, die aus 25 Gew.-Teilen Cyclohexanol, 65 Gew.-Teilen einer Mineralölfraktion vom Siedepunkt 210 bis 280 °C und 10 Gew.-Teilen des Anlagerungsproduktes von 40 Mol Ethylenoxid an 1 Mol Ricinusöl besteht. Durch Eingießen und feines Verteilen der Lösung in Wasser erhält man eine wäßrige Dispersion.

V. 80 Gew.-Teile der Verbindung Nr. 1 werden mit 3 Gew.-Teilen des Natriumsalzes der Diisobutylnaphthalin-$\alpha$-sulfonsäure, 10 Gew.-Teilen des Natriumsalzes einer Ligninsulfonsäure aus einer Sulfitablauge und 7 Gew.-Teilen pulverförmigem Kieselsäuregel gut vermischt und in einer Hammermühle vermahlen. Durch feines Verteilen der Mischung in Wasser erhält man eine Spritzbrühe.

VI. 3 Gew.-Teile der Verbindung Nr. 5 werden mit 97 Gew.-Teilen feinteiligem Kaolin innig vermischt. Man erhält auf diese Weise ein Stäubemittel, das 3 Gew. % des Wirkstoffs enthält.

VII. 30 Gew.-Teile der Verbindung Nr. 6 werden mit einer Mischung aus 92 Gew.-Teilen pulverförmigem Kieselsäuregel und 8 Gew.-Teilen Paraffinöl, das auf die Oberfläche dieses Kieselsäuregels gesprüht wurde, innig vermischt. Man erhält auf diese Weise eine Aufbereitung der Wirkstoffs mit guter Haftfähigkeit.

VIII. 40 Gew.-Teile der Verbindung Nr. 1 werden mit 10 Gew.-Teilen Natriumsalz eines Phenolsulfonsäure-harnstoff-formaldehyd-Kondensates, 2 Gew.-Teilen Kieselgel und 48 Gew.-Teilen Wasser innig vermischt. Man erhält eine stabile wäßrige Dispersion. Durch Verdünnen mit Wasser erhält man eine wäßrige Dispersion.

IX. 20 Gew.-Teile der Verbindung Nr. 1 werden mit 2 Gew.-Teilen Calciumsalz der Dodecylbenzolsulfonsäure, 8 Gew.-Teilen Fettalkoholpolyglykolether, 2 Gew.-Teilen Natriumsalz eines Phenolsulfonsäure-harnstoff-formaldehyd-Kondensats und 68 Gew.-Teilen eines paraffinischen Mineralöls innig vermischt. Man erhält eine stabile ölige Dispersion.

Die erfindungsgemäßen Mittel können in diesen Anwendungsformen auch zusammen mit anderen Wirkstoffen vorliegen, wie z.B. Herbiziden, Insektiziden, Wachstumsregulatoren und Fungiziden, oder auch mit Düngemitteln vermischt und ausgebracht werden. Beim Vermischen mit Fungiziden erhält man dabei in vielen Fällen eine Vergrößerung des fungiziden Wirkungsspektrums.

Anwendungsbeispiele

Als Vergleichswirkstoffe wurden die Verbindung
2-n-Butyl-3-(4-methylphenyl)-1-(3-pyridinyl)-2-propen-1-on (C),
2-n-Butyl-3-(4-fluorphenyl)-1-(3-pyridinyl)-2-propen-1-ol (A),
2-n-Butyl-3-(4-methylphenyl)-1-(3-pyridinyl)-2-propen-1-ol (B)
- bekannt aus EP-214 566 - benutzt.

Anwendungsbeispiel 1

Wirksamkeit gegen Weizenmehltau

Blätter von in Töpfen gewachsenen Weizenkeimlingen der Sorte "Frühgold" wurden mit wäßriger Spritzbrühe, die 80 % Wirkstoff und 20 % Emulgiermittel in der Trockensubstanz enthielten, besprüht und 24 Stunden nach dem Antrocknen des Spritzbelages mit Oidien (Sporen) des Weizenmehltaus (Erysiphe graminis var. tritici) bestäubt. Die Versuchspflanzen wurden anschließend im Gewächshaus bei Temperaturen zwischen 20 und 22 °C und 75 bis 80 % relativer Luftfeuchtigkeit aufgestellt. Nach 7 Tagen wurde das Ausmaß der Mehltauentwicklung ermittelt.

Das Ergebnis zeigt, daß die Wirkstoffe 1 und 5 bei der Anwendung als 0,025 und 0,006 %ige (Gew.%) Spritzbrühe eine bessere fungizide Wirkung zeigen (97 %) als die bekannten Wirkstoffe A, B und C (70 %).

Anwendungsbeispiel 2

Wirksamkeit gegen Gurkenmehltau

Blätter von in Töpfen gewachsenen Gurkenkeimlingen der Sorte "Chinesische Schlange" wurden im Zweiblattstadium mit einer Sporensuspension des Gurkenmehltaus besprüht. Nach 3 Tagen wurden die Versuchspflanzen mit wäßriger Spritzbrühe, die 80 % Wirkstoff und 20 % Emulgiermittel in der Trockensubstanz enthielt bis zur Tropfnäße behandelt. Nach dem Antrocknen des Spritzbelages wurden sie anschließend im Gewächshaus bei Temperaturen zwischen 20 und 22 °C und 70 - 80 % relativer Luftfeuchtigkeit aufgestellt. Zur Beurteilung der Wirksamkeit der neuen Stoffe wurde das Ausmaß der Pilzentwicklung 21

Tage nach der Behandlung ermittelt.

Das Ergebnis zeigt, daß die Wirkstoffe 1, 5 und 6 bei der Anwendung als 0,0125 und 0,006 %ige Spritzbrühen eine bessere fungizide Wirkung zeigen (90 %) als die bekannten Wirkstoffe A und B (10 %).

Anwendungsbeispiel 3

Wirksamkeit gegen Plasmopara viticola

Blätter von Topfreben der Sorte "Müller Thurgau" wurden mit wäßriger Spritzbrühe, die 80 % Wirkstoff und 20 % Emulgiermittel in der Trockensubstanz enthielt, besprüht. Um die Wirkungsdauer der Wirkstoffe beurteilen zu können, wurden die Pflanzen nach dem Antrocknen des Spritzbelages 8 Tage im Gewächshaus aufgestellt. Erst dann wurden die Blätter mit einer Zoosporenaufschwemmung von Plasmopara viticola (Rebenperonospora) infiziert. Danach wurden die Reben zunächst für 48 Stunden in einer wasserdampfgesättigten Kammer bei 24°C und anschließend für 5 Tage in einem Gewächshaus mit Temperaturen zwischen 20 und 30°C aufgestellt. Nach dieser Zeit wurden die Pflanzen zur Beschleunigung des Sporangienträgerausbruches abermals für 16 Stunden in der feuchten Kammer aufgestellt. Dann erfolgte die Beurteilung des Ausmaßes des Pilzausbruches auf den Blattunterseiten.

Das Ergebnis zeigt, daß die Wirkstoffe 1, 5 und 6 bei der Anwendung als 0,05 %ige Spritzbrühen eine bessere fungizide Wirkung zeigen (97 %) als die bekannten Wirkstoffe A, B und C (50 %).

Anwendungsbeispiel 4

Wirksamkeit gegen Pyrenophora teres

Gerstenkeimlinge der Sorte "Igri" wurden im Zweiblattstadium mit wäßrigen Suspensionen, die 80 % Wirkstoff und 20 % Emulgator in der Trockensubstanz enthielten, tropfnaß gespritzt. Nach 24 Stunden wurden die Pflanzen mit einer Sporensuspension des Pilzes Pyrenophora teres inokuliert und für 48 Stunden in eine Klimakammer mit hoher Luftfeuchtigkeit bei 18°C gestellt. Anschließend wurden die Pflanzen im Gewächshaus bei 20-22°C und 70 % relativer Luftfeuchtigkeit für weitere 5 Tage kultiviert. Dann wurde das Ausmaß der Symptomentwicklung ermittelt.

Das Ergebnis zeigt, daß die Wirkstoffe 1, 5 und 6 bei der Anwendung als 0,05 %ige Spritzbrühe eine bessere fungizide Wirkung zeigen (97 %) als die bekannten Wirkstoffe A, B und C (50 %).

**Patentansprüche**

**1.** 3-substituiertes Pyridin der Formel

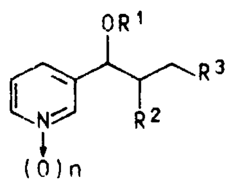

(I)

in der

$R^1$ Wasserstoff, $C_1$-$C_8$-Alkyl, $C_3$-$C_8$-Alkenyl, $C_3$-$C_8$-Alkinyl, $C_2$-$C_{12}$-Acyl, $C_2$-$C_{12}$-Halogenacyl, ungesättigtes $C_3$-Acyl, gegebenenfalls durch $C_1$-$C_4$-Alkyl, $C_1$-$C_4$-Alkoxy, Phenyl, Acetoxy, $C_1$-$C_4$-Halogenalkyl, Halogen, Nitro substituiertes Benzoyl, gegebenenfalls durch $C_1$-$C_4$-Alkyl, $C_1$-$C_4$-Alkoxy, $C_1$-$C_4$-Halogenalkyl, Halogen, Cyan, Nitro substituiertes Benzyl;

$R^2$ Wasserstoff, $C_1$-$C_{12}$-Alkyl, $C_3$-$C_{12}$-Cycloalkyl, $C_4$-$C_{20}$-Cycloalkylalkyl, $C_4$-$C_{20}$-Alkylcycloalkyl.

$R^3$ den Rest

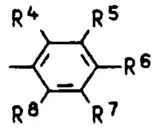 oder 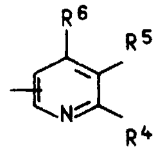 bedeutet,

wobei $R^4$ bis $R^8$ unabhängig voneinander Wasserstoff, $C_1$-$C_4$-Alkyl, $C_1$-$C_4$-Alkoxy, $C_1$-$C_4$-Halogenalkyl, $C_2$-$C_4$-Alkoxycarbonyl, $C_2$-$C_4$-Dialkylamino, Phenyl, Hydroxy oder Halogen bedeuten und $R^4$ und $R^5$ oder $R^5$ und $R^6$ gemeinsam zusätzlich die Gruppe -CH = CH-CH = CH-bedeuten, n den Wert 0 oder 1 hat und seine für Pflanzen verträglichen Salze.

**2.** Verfahren zur Herstellung eines Pyridins der Formel I gemäß Anspruch 1, in der $R^1$ Wasserstoff bedeutet, dadurch gekennzeichnet, daß man ein Pyridylketon der Formel III oder einen Pyridylalkohol der Formel IV,

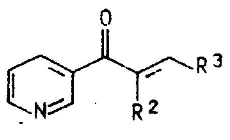 (III) 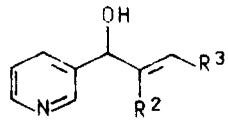 (IV),

in denen $R^2$ und $R^3$ die in Anspruch 1 genannten Bedeutungen haben, mit einem Reduktionsmittel reduziert und die so erhaltene Verbindung gegebenenfalls mit einem Alkylierungs- oder Acylierungsmittel der Formel II,

$R^1$-X (II)

in der $R^1$ die in Anspruch 1 genannten Bedeutungen außer Wasserstoff hat und X eine nucleofuge Gruppe bedeutet, umsetzt.

**3.** Verfahren zur Herstellung eines Pyridins der Formel I gemäß Anspruch 1, in der $R^1$ Wasserstoff bedeutet, dadurch gekennzeichnet, daß man eine Organometallverbindung der Formel V,

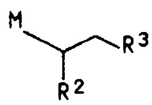 (V)

in der $R^2$ und $R^3$ die unter Anspruch 1 genannten Bedeutungen haben und M für Lithium oder einen der Reste MgCl oder MgBr steht, mit Nicotinaldehyd umsetzt.

**4.** Fungizides Mittel, enthaltend einen inerten Trägerstoff und eine fungizide Menge eines 3-substituierten Pyridins der Formel

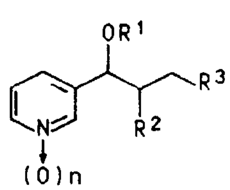 (I)

in der

$R^1$ Wasserstoff, $C_1$-$C_8$-Alkyl, $C_3$-$C_8$-Alkenyl, $C_3$-$C_8$-Alkinyl, $C_2$-$C_{12}$-Acyl, $C_2$-$C_{12}$-Halogenacyl, ungesättigtes $C_3$-Acyl, gegebenenfalls durch $C_1$-$C_4$-Alkyl, $C_1$-$C_4$-Alkoxy, Phenyl, Acetoxy, $C_1$-$C_4$-Halogenalkyl, Halogen, Nitro substituiertes Benzovl, gegebenenfalls durch $C_1$-$C_4$-Alkyl, $C_1$-

$C_4$-Alkoxy, $C_1$-$C_4$-Halogenalkyl, Halogen, Cyan Nitro substituiertes Benzyl;

$R^2$ Wasserstoff, $C_1$-$C_{12}$-Alkyl, $C_3$-$C_{12}$-Cycloalkyl, $C_4$-$C_{20}$-Cycloalkylalkyl, $C_4$-$C_{20}$-Alkylcycloalkyl,

$R^3$ den Rest

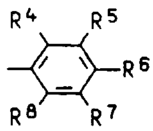 oder 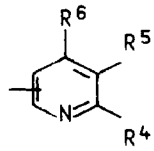 bedeutet,

wobei $R^4$ bis $R^8$ unabhängig voneinander Wasserstoff, $C_1$-$C_4$-Alkyl, $C_1$-$C_4$-Alkoxy, $C_1$-$C_4$-Halogenalkyl, $C_2$-$C_4$-Alkoxycarbonyl, $C_2$-$C_4$-Dialkylamino, Phenyl, Hydroxy oder Halogen bedeuten und $R^4$ und $R^5$ oder $R^5$ und $R^6$ gemeinsam zusätzlich die Gruppe -CH = CH-CH = CH- bedeuten, n den Wert 0 oder 1 hat oder sein für Pflanzen verträgliches Salz.

**5.** Verfahren zur Bekämpfung von Pilzen, dadurch gekennzeichnet, daß man die Pilze oder die vom Pilzbefall bedrohten Materialien, Pflanzen, Böden oder Saatgüter behandelt mit einer fungizid wirksamen Menge eines 3-substituierten Pyridins der Formel

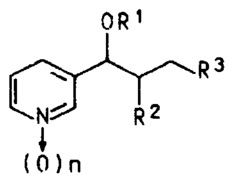

(I)

in der

$R^1$ Wasserstoff, $C_1$-$C_8$-Alkyl, $C_3$-$C_8$-Alkenyl, $C_3$-$C_8$-Alkinyl, $C_2$-$C_{12}$-Acyl, $C_2$-$C_{12}$-Halogenacyl, ungesättigtes $C_3$-Acyl, gegebenenfalls durch $C_1$-$C_4$-Alkyl, $C_1$-$C_4$-Alkoxy, Phenyl, Acetoxy, $C_1$-$C_4$-Halogenalkyl, Halogen, Nitro substituiertes Benzoyl, gegebenenfalls durch $C_1$-$C_4$-Alkyl, $C_1$-$C_4$-Alkoxy, $C_1$-$C_4$-Halogenalkyl, Halogen, Cyan, Nitro substituiertes Benzyl;

$R^2$ Wasserstoff, $C_1$-$C_{12}$-Alkyl, $C_3$-$C_{12}$-Cycloalkyl, $C_4$-$C_{20}$-Cycloalkylalkyl, $C_4$-$C_{20}$-Alkylcycloalkyl,

$R^3$ den Rest

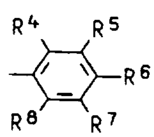 oder 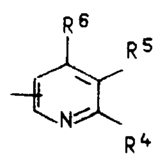 bedeutet,

wobei $R^4$ bis $R^8$ unabhängig voneinander Wasserstoff, $C_1$-$C_4$-Alkyl, $C_1$-$C_4$-Alkoxy, $C_1$-$C_4$-Halogenalky, $C_2$-$C_4$-Alkoxycarbonyl, $C_2$-$C_4$-Dialkylamino, Phenyl, Hydroxy oder Halogen bedeuten und $R^4$ und $R^5$ oder $R^5$ und $R^6$ gemeinsam zusätzlich die Gruppe -CH = CH-CH = CH- bedeuten, n den Wert 0 oder 1 hat oder seinem für Pflanzen verträglichen Salz.

**6.** 2-n-Butyl-3-(4-fluorphenyl)-1-(3-pyridinyl)-propan-1-ol.

**7.** 2-n-Butyl-3-(4-methylphenyl)-1-(3-pyridinyl)-propan-1-ol.

**8.** 2-n-Butyl-3-(4-tertiär-butylphenyl)-1-(3-pyridinyl)-propan-1-ol.

## Claims

**1.** A 3-substituted pyridine of the formula

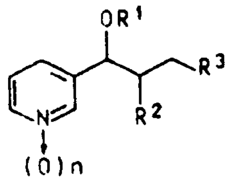

(I)

where

$R^1$ is hydrogen, $C_1$-$C_8$-alkyl, $C_3$-$C_8$-alkenyl, $C_3$-$C_8$-alkynyl, $C_2$-$C_{12}$-acyl, $C_2$-$C_{12}$-haloacyl or unsaturated $C_3$-acyl, or is benzoyl which is unsubstituted or substituted by $C_1$-$C_4$-alkyl, $C_1$-$C_4$-alkoxy, phenyl, acetoxy, $C_1$-$C_4$-haloalkyl, halogen or nitro, or is benzyl which is unsubstituted or substituted by $C_1$-$C_4$-alkyl, $C_1$-$C_4$-alkoxy, $C_1$-$C_4$-haloalkyl, halogen, cyano or nitro;

$R^2$ is hydrogen, $C_1$-$C_{12}$-alkyl, $C_3$-$C_{12}$-cycloalkyl, $C_4$-$C_{20}$-cycloalkylalkyl or $C_4$-$C_{20}$-alkylcycloalkyl

$R^3$ is the radical

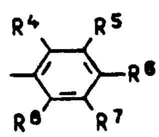

or

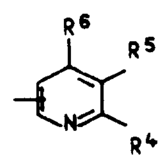

where $R^4$ to $R^8$, independently of one another, are each hydrogen, $C_1$-$C_4$-alkyl, $C_1$-$C_4$-alkoxy, $C_1$-$C_4$-haloalkyl, $C_2$-$C_4$-alkoxycarbonyl, $C_2$-$C_4$-dialkylamino, phenyl, hydroxy or halogen, and $R^4$ and $R^5$ or $R^5$ and $R^6$ together may furthermore be the group -CH = CH-CH = CH, and

n is 0 or 1, and its plant-tolerated salts.

**2.** A process for the preparation of a pyridine of the formula I as claimed in claim 1, $R^1$ being hydrogen, wherein a pyridyl ketone of the formula III or a pyridyl alcohol of the formula IV,

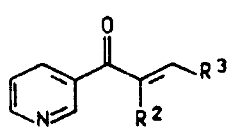

(III)

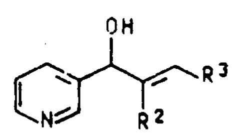

(IV)

where $R^2$ and $R^3$ have the meanings given in claim 1, is reduced with a reducing agent, and the compound thus obtained is, if desired, reacted with an alkylating or acylating agent of the formula II

$R^1$-X (II)

where $R^1$ has the meanings given in claim 1, with the exception of hydrogen, and X is a nucleofugic group.

**3.** A process for the preparation of a pyridine of the formula I as claimed in claim 1, $R^1$ being hydrogen, wherein an organometallic compound of the formula V,

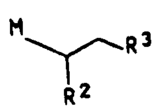

(V)

where $R^2$ and $R^3$ have the meanings given in claim 1 and M is lithium or one of the radicals MgCl or MgBr, is reacted with nicotinaldehyde.

**4.** A fungicidal agent containing an inert carrier and a fungicidal amount of a 3-substituted pyridine of the formula

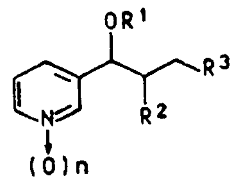

(I)

where

$R^1$ is hydrogen, $C_1$-$C_8$-alkyl, $C_3$-$C_8$-alkenyl, $C_3$-$C_8$-alkynyl, $C_2$-$C_{12}$-acyl, $C_2$-$C_{12}$-haloacyl, unsaturated $C_3$-acyl, benzoyl which is unsubstituted or substituted by $C_1$-$C_4$-alkyl, $C_1$-$C_4$-alkoxy, phenyl, acetoxy, $C_1$-$C_4$-haloalkyl, halogen or nitro, or is benzyl which is unsubstituted or substituted by $C_1$-$C_4$-alkyl, $C_1$-$C_4$-alkoxy, $C_1$-$C_4$-haloalkyl, halogen, cyano or nitro;

$R^2$ is hydrogen, $C_1$-$C_{12}$-alkyl, $C_3$-$C_{12}$-cycloalkyl, $C_4$-$C_{20}$-cycloalkylalkyl or $C_4$-$C_{20}$alkylcycloalkyl

$R^3$ is the radical

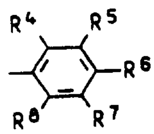

or

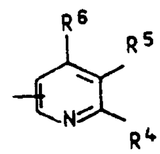

where $R^4$ to $R^8$, independently of one another, are each hydrogen, $C_1$-$C_4$-alkyl, $C_1$-$C_4$-alkoxy, $C_1$-$C_4$-haloalkyl, $C_2$-$C_4$-alkoxycarbonyl, $C_2$-$C_4$-dialkyl-amino, phenyl, hydroxy or halogen, and $R^4$ and $R^5$ or $R^5$ and $R^6$ together may furthermore be the group -CH = CH-CH = CH-,

n is 0 or 1, or a plant-tolerated salt thereof.

**5.** A method for controlling fungi, wherein the fungi, or the materials, plants, soils or seed threatened by fungal attack are treated with a fungicidally effective amount of a 3-substituted pyridine of the formula

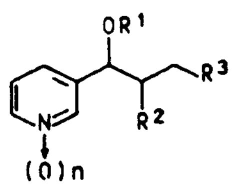

(I)

where

$R^1$ is hydrogen, $C_1$-$C_8$-alkyl, $C_3$-$C_8$-alkenyl, $C_3$-$C_8$-alkynyl, $C_2$-$C_{12}$-acyl, $C_2$-$C_{12}$-haloacyl, unsaturated $C_3$-acyl, benzoyl which is unsubstituted or substituted by $C_1$-$C_4$-alkyl, $C_1$-$C_4$-alkoxy, phenyl, acetoxy. $C_1$-$C_4$-haloalkyl, halogen or nitro, or is benzyl which is unsubstituted or substituted by $C_1$-$C_4$-alkyl, $C_1$-$C_4$-alkoxy, $C_1$-$C_4$-haloalkyl, halogen, cyano or nitro;

$R^2$ is hydrogen, $C_1$-$C_{12}$-alkyl, $C_3$-$C_{12}$-cycloalkyl, $C_4$-$C_{20}$-cycloalkylalkyl or $C_4$-$C_{20}$-alkylcycloalkyl,

$R^3$ is the radical

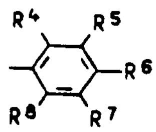

or

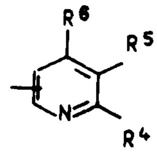

where $R^4$ to $R^8$, independently of one another, are each hydrogen, $C_1$-$C_4$-alkyl, $C_1$-$C_4$-alkoxy, $C_1$-$C_4$-haloalkyl, $C_2$-$C_4$-alkoxycarbonyl, $C_2$-$C_4$-dialkylamino, phenyl, hydroxy or halogen, and $R^4$ and $R^5$ or $R^5$ and $R^6$ together may furthermore be the group -CH = CH-CH = CH-,

n is 0 or 1, or a plant-tolerated salt thereof.

**6.** 2-n-Butyl-3-(4-fluorophenyl)-1-(3-pyridinyl)-propan-1-ol.

**7.** 2-n-Butyl-3-(4-methylphenyl)-1-(3-pyridinyl)-propan-1-ol.

**8.** 2-n-Butyl-3-(4-tert.-butylphenyl)-1-(3-pyridinyl)-propan-1-ol.

**Revendications**

**1.** Pyridine substituée en 3, de la formule

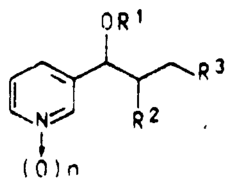

(I)

dans laquelle

$R^1$ représente hydrogène, alkyle en C1-C8, alcényle en C3-C8, alcynyle en C3-C8, acyle en C2-C12, halogénoacyle en C2-C12, acyle en C3 insaturé benzoyle éventuellement substitué par alkyle en C1-C4, alcoxy en C1-C4, phényle, acétoxy, halogènalkyle en C1-C4, halogène, nitro, benzoyle éventuellement substitué par alkyle en C1-C4, alcoxy en C1-C4, halogènalkyle en C1-C4, halogène, cyano, nitro ;

$R^2$, hydrogène, alkyle en C1-C12, cycloalkyle en C3-C12, cycloalkylealkyle en C4-C20, alkylcycloalkyle en C4-C20 ;

$R^3$, le reste

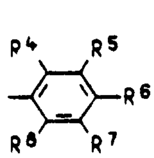

ou

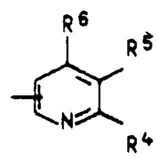

$R^4$ à $R^8$ représentant, indépendamment les uns des autres, hydrogène, alkyle en C1-C4, alcoxy en C1-C4, halogènalkyle en C1-C4, alcoxycarbonyle en C2-C4, dialkylamino, phényle, hydroxy ou halogène et $R^4$ et $R^5$ ou $R^5$ et $R^6$ représentent ensemble additionnellement, le groupe -CH = CH-CH = CH-

n, a la valeur 0 ou 1

et ses sels compatibles pour les plantes.

**2.** Procédé de préparation d'une pyridine de la formule I selon la revendication 1 dans laquelle $R^1$ représente l'hydrogène, caractérisé par le fait que l'on réduit une pyridylcétone de la formule III ou un alcool pyridylique de la formule IV

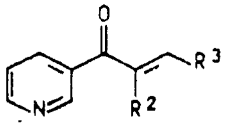
(III)

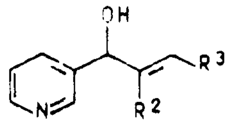
(IV),

dans lesquelles $R^2$ et $R^3$ ont la signification donnée dans la revendication 1, avec un agent de réduction et que l'on fait réagir le composé ainsi obtenu, éventuellement avec un agent d'alkylation ou d'acylation de la formule II

$R^1$-X (II)

dans laquelle $R^1$ a la signification donnée dans la revendication 1, à l'exception de l'hydrogène et X représente un groupe nucléofuge.

**3.** Procédé de préparation d'une pyridine de la formule I selon la revendication 1 dans laquelle $R^1$ représente l'hydrogène, caractérisé par le fait que l'on fait réagir avec de l'aldéhyde nicotinique, un composé organométallique de la formule V

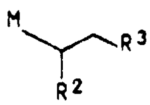
(V)

dans laquelle $R^2$ et $R^3$ ont les significations données dans la revendication 1 et M est mis pour lithium ou un des restes Mgcl ou MgBr.

**4.** Fongicide contenant un support inerte et une quantité fongicide d'une pyridine substituée en 3, de la formule

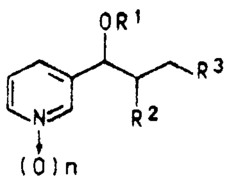
(I)

dans laquelle

- $R^1$ représente hydrogène, alkyle en C1-C8, alcényle en C3-C8, alcynyle en C3-C8, acyle en C2-C12, halogénoacyle en C2-C12, acyle en C3 insaturé benzoyle éventuellement substitué par alkyle en C1-C4, alcoxy en C1-C4, phényle, acétoxy, halogènalkyle en C1-C4, halogène, nitro, benzoyle éventuellement substitué par alkyle en C1-C4, alcoxy en C1-C4, halogènalkyle en C1-C4, halogène, cyano, nitro ;
- $R^2$, hydrogène, alkyle en C1-C12, cycloalkyle en C3-C12, cycloalkylealkyle en C4-C20, alkylcycloalkyle en C4-C20 ;
- $R^3$, le reste

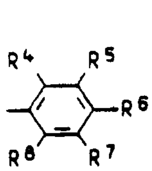
ou
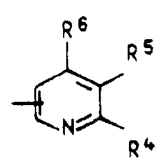

$R^4$ à $R^8$ représentant, indépendamment les uns des autres, hydrogène, alkyle en C1-C4,

alcoxy en C1-C4, halogènalkyle en C1-C4, alcoxycarbonyle en C2-C4, dialkylamino, phényle, hydroxy ou halogène et $R^4$ et $R^5$ ou $R^5$ et $R^6$ représentent ensemble additionnellement, le groupe -CH = CH-CH = CH-

n, a la valeur 0 ou 1

et ses sels compatibles pour les plantes.

**5.** Procédé pour la lutte contre les champignons, caractérisé par le fait que l'on traite les champignons ou les matériaux, plantes, sols ou semences menacés d'une attaque par les champignons, avec une quantité efficace du point de vue fongicide d'une pyridine substituée en 3, de la formule

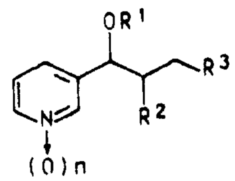

(I)

dans laquelle

$R^1$ représente hydrogène, alkyle en C1-C8, alcényle en C3-C8, alcynyle en C3-C8, acyle en C2-C12, halogénoacyle en C2-C12, acyle en C3 insaturé benzoyle éventuellement substitué par alkyle en C1-C4, alcoxy en C1-C4, phényle, acétoxy, halogènalkyle en C1-C4, halogène, nitro, benzoyle éventuellement substitué par alkyle en C1-C4, alcoxy en C1-C4, halogènalkyle en C1-C4, halogène, cyano, nitro ;

$R^2$, hydrogène, alkyle en C1-C12, cycloalkyle en C3-C12, cycloalkylealkyle en C4-C20, alkylcycloalkyle en C4-C20 ;

$R^3$, le reste

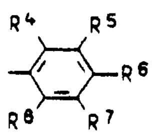

ou

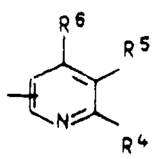

$R^4$ à $R^8$ représentant, indépendamment les uns des autres, hydrogène, alkyle en C1-C4, alcoxy en C1-C4, halogènalkyle en C1-C4, alcoxycarbonyle en C2-C4, dialkylamino, phényle, hydroxy ou halogène et $R^4$ et $R^5$ ou $R^5$ et $R^6$ représentent ensemble additionnellement, le groupe -CH = CH-CH = CH-

n, a la valeur 0 ou 1

et ses sels compatibles pour les plantes.

**6.** 2-n-butyl-3-(4-fluorophényl)-1-(3-pyridinyl)-propan-1-ol.

**7.** 2-n-butyl-3-(4-méthylphényl)-1-(3-pyridinyl)-propan-1-ol.

**8.** 2-n-butyl-3-(4-tertiaire-butylphényl)-1-(3-pyridinyl)-propan-1-ol.